(19) Europäisches Patentamt
European Patent Office
Office européen des brevets

(11) Veröffentlichungsnummer: **0 011 757**
**A1**

(12) **EUROPÄISCHE PATENTANMELDUNG**

(21) Anmeldenummer: **79104438.1**

(51) Int. Cl.³: **C 07 D 265/26**

(22) Anmeldetag: **12.11.79**

(30) Priorität: **25.11.78 DE 2851099**

(43) Veröffentlichungstag der Anmeldung:
**11.06.80 Patentblatt 80/12**

(84) Benannte Vertragsstaaten:
**BE CH DE FR GB IT**

(71) Anmelder: **BASF Aktiengesellschaft**
**Carl-Bosch-Strasse 38**
**D-6700 Ludwigshafen(DE)**

(72) Erfinder: **Schneider, Dieter, Dr.**
**Neumannstrasse 18**
**D-6700 Ludwigshafen(DE)**

(72) Erfinder: **Scheuermann, Horst, Dr.**
**Bexbacher Strasse 41**
**D-6700 Ludwigshafen(DE)**

(54) **6,8-Dinitroisatosäureanhydrid und Verfahren zu seiner Herstellung.**

(57) Das neue 6,8-Dinitroisatosäureanhydrid und ein Verfahren zu seiner Herstellung durch Nitrierung von Isatosäureanhydrid mit einem Nitriergemisch bestimmter Zusammensetzung bei -20 bis +80°C.

Das nach dem Verfahren der Erfindung herstellbare neue 6,8-Dinitroisatosäureanhydrid ist ein wertvoller Ausgangsstoff für die Herstellung von Farbstoffen, Pharmazeutika und Schädlingsbekämpfungsmitteln.

EP 0 011 757 A1

## 6,8-Dinitroisatosäureanhydrid und Verfahren zu seiner Herstellung

Die Erfindung betrifft das neue 6,8-Dinitroisatosäureanhydrid und ein Verfahren zu seiner Herstellung durch Nitrierung von Isatosäureanhydrid mit einem Nitriergemisch bestimmter Zusammensetzung bei -20 bis +80°C.

Es ist aus der deutschen Offenlegungsschrift 24 22 239 bekannt, daß man Isatosäureanhydrid mit Salpetersäure und Oleum in einem Verhältnis von 0,5 bis 20 Mol Schwefeltrioxid je Mol Salpetersäure in Gegenwart von organischen Lösungsmitteln bei einer Temperatur von -20 bis +80°C nitriert und in einem zweiten Schritt die bei der Nitrierung gebildete saure Phase des zweiphasigen Reaktionsgemisches mit Ammoniak umsetzt. Die Umsetzung wird durch die folgenden Formeln wiedergegeben:

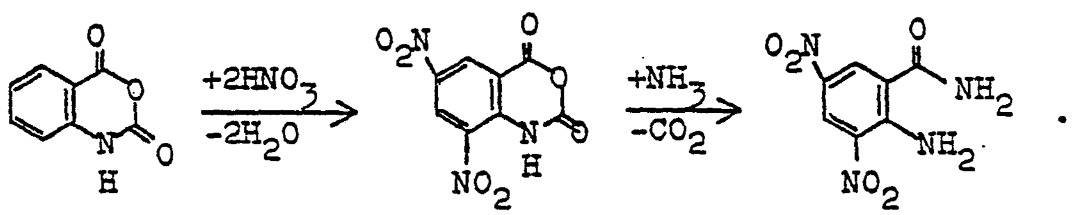


Der Endstoff 2-Amino-3,5-dinitrobenzamid wird einbadig in 2 Arbeitsschritten ohne Aufarbeitung des Reaktionsgemisches der Nitrierung und Isolierung eines etwa gebildeten, hypo-

thetischen Dinitroisatosäureanhydrids hergestellt. Die Beschreibung bringt weder charakteristische Daten oder Eigenschaften des Dinitroisatosäureanhydrids, noch gibt sie einen Weg zur Isolierung dieses Stoffes an. Alle Beispiele zeigen lediglich Nitriergemische, die ohne Isolierung eines Dinitroisatosäureanhydrids sofort weiterverarbeitet werden. Die oben angegebene Formelgleichung wird daher in der Veröffentlichung nur als Hypothese erwähnt. Das beschriebene Herstellungsverfahren für diese Nitriergemische ist im Hinblick auf Isolierung des Endstoffs in hoher Ausbeute und Reinheit, einfachen, wirtschaftlichen und sicheren Betrieb, unbefriedigend.

Es wurde das neue 6,8-Dinitroisatosäureanhydrid gefunden.

Weiterhin wurde gefunden, daß man 6,8-Dinitroisatosäureanhydrid durch Nitrierung von Isatosäureanhydrid vorteilhaft erhält, wenn man Isatosäureanhydrid mit Salpetersäure in Gegenwart von 90- bis 100-gewichtsprozentiger Schwefelsäure bei einer Temperatur von -20 bis +80°C nitriert.

Die Umsetzung kann durch die folgenden Formeln wiedergegeben werden:

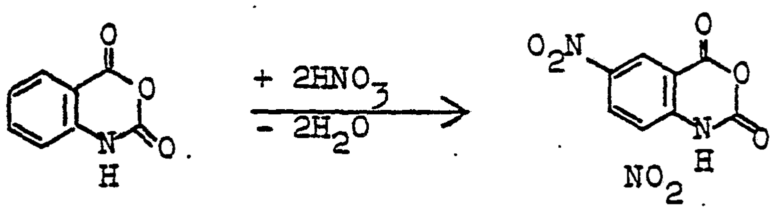


Im Hinblick auf das bekannte Verfahren liefert das Verfahren nach der Erfindung auf einfachem und wirtschaftlichem Wege das neue 6,8-Dinitroisatosäureanhydrid in guter Ausbeute und Reinheit. In der Regel verwendet man keine organischen Lösungsmittel und erspart sich so ebenfalls den technisch komplizierteren Betrieb eines 2-Phasensystems.

Das erfindungsgemäße Gemisch kann besser und rascher durch Rühren homogen verteilt und eine ungenügende Reaktion bzw. anders verlaufende Reaktion infolge schlechter Durchmischung vermieden werden. Große Mengen an Oleum bzw. Schwefeltrioxid werden nicht benötigt, Schwierigkeiten bezüglich der Sicherheit von Personal und Anlagen, des Umweltschutzes sowie der Regelung und Kontrolle des Betriebs werden wesentlich verringert. Das bekannte Verfahren bedarf andererseits besonders eingehender Regelung, um eine spontane Reaktionsbeschleunigung, Zersetzung der Reaktionsgemische und erhöhte Bildung von Nebenstoffen unter Kontrolle zu halten; das erfindungsgemäße Verfahren ist daher betriebssicherer und umweltfreundlicher. Alle diese vorteilhaften Ergebnisse sind im Hinblick auf den Stand der Technik überraschend.

Man nitriert mit Salpetersäure, vorteilhaft konzentrierter oder rauchender Salpetersäure, in Gegenwart von Schwefelsäure, die kein freies $SO_3$ enthält. Im allgemeinen verwendet man eine 85- bis 100-, vorzugsweise 95- bis 100-gewichtsprozentige Salpetersäure und eine 90- bis 99,9-, vorzugsweise 97- bis 99,9-gewichtsprozentige Schwefelsäure. In der Regel ergänzt Wasser die vorgenannten Prozentangaben der Säuren auf 100 Prozent. Zweckmäßig wählt man in dem Gemisch von Salpetersäure und Schwefelsäure, der Nitriersäure, ein Verhältnis von 2 bis 15, insbesondere von 4 bis 6 Mol Schwefelsäure je Mol Salpetersäure. In der Regel verwendet man von 2 bis 10 Mol, vorzugsweise von 2 bis 5 Mol Salpetersäure je Mol Ausgangsstoff. Anstelle von Salpetersäure können auch Stoffe, die diese Säure im Reaktionsgemisch bilden, z.B. anorganische Nitrate wie Natrium- oder Kaliumnitrat, in entsprechenden Mengen zur Anwendung gelangen. Gegebenenfalls verwendet man Harnstoff als Nitrierkatalysator, zweckmäßig in einer Menge von 0,05 bis 10, vorzugsweise von 0,1 bis 5 Gewichtsprozent, bezogen

auf Isatosäureanhydrid.

Die Umsetzung wird bei einer Temperatur von -20°C bis +80°C, vorzugsweise von 0°C bis 50°C, insbesondere von 5 bis 30°C, drucklos oder unter Druck, diskontinuierlich oder kontinuierlich durchgeführt. Lösungsmedium ist die Säure bzw. das Säuregemisch und Wasser im allgemeinen selbst. Doch kann man gegebenenfalls die Reaktion in Gegenwart von unter den Reaktionsbedingungen inerten, organischen Lösungsmitteln durchführen. Als organische Lösungsmittel kommen z.B. in Frage: Halogenkohlenwasserstoffe, insbesondere Chlorkohlenwasserstoffe, z.B. Tetrachloräthylen, Amylchlorid, Cyclohexylchlorid, Dichlorpropan, Methylenchlorid, Dichlorbutan, Isopropylbromid, n-Propylbromid, Butylbromid, Chloroform, Tetrachlorkohlenstoff, Tetrachloräthan, Trichloräthan, Trichloräthylen, Pentachloräthan, cis-Dichloräthylen, o-, m-, p-Difluorbenzol, 1,2-Dichloräthan, 1,1-Dichloräthan, n-Propylchlorid, 1,2-cis-Dichloräthylen, n-Butylchlorid, 2-, 3- und iso-Butylchlorid, Fluorbenzol, o-, p- und m-Dichlorbenzol, o-, p-, m-Dibrombenzol, o-, m-, p-Chlortoluol, 1,2,4-Trichlorbenzol, 1,10-Dibromdekan, 1,4-Dibrombutan; aliphatische oder cycloaliphatische Kohlenwasserstoffe, z.B. Heptan, Nonan, Pinan, Benzinfraktionen innerhalb des Siedepunktintervalls von 70 bis 190°C, Cyclohexan, Methylcyclohexan, Petroläther, Dekalin, Hexan, Ligroin, 2,2,4-Trimethylpentan, 2,2,3-Trimethylpentan, 2,3,3-Trimethylpentan, Octan; und entsprechende Gemische. In solchen weniger zweckmäßigen Fällen verwendet man das organische Lösungsmittel in einer Menge von 0 bis 5 000 Gewichtsprozent, vorzugsweise von 0 bis 2 000 Gewichtsprozent, bezogen auf Ausgangsstoff.

Die Reaktion kann wie folgt durchgeführt werden: Ein Gemisch von Ausgangsstoff, Nitriersäure und gegebenenfalls Harnstoff in Abwesenheit organischer Lösungsmittel wird während 2 bis 10 Stunden bei der Reaktionstemperatur gehalten. Vorteilhaft wird das Ausgangsgemisch durch langsame Eingabe des Ausgangsstoffs zum Gemisch der anderen Komponenten, z.B. während 0,5 bis 1,5 Stunden, hergestellt. Zweckmäßig wird zuerst bei 20 bis 30°C in die konzentrierte Schwefelsäure die konzentrierte Salpetersäure langsam eingegeben. Danach kann man bei 20 bis 30°C Harnstoff zusetzen, um Nitrosoverbindungen zu zerstören. Man kühlt dann zweckmäßig auf 5 bis 20°C ab, bevorzugt 12 bis 18°C, und trägt in das Gemisch Isatosäureanhydrid unter Kühlung ein. Danach rührt man das Gemisch bei 5 bis 25°C, bevorzugt 15 bis 22°C, 1 bis 8 Stunden, insbesondere 2 bis 4 Stunden, nach. Dann wird der Endstoff in üblicher Weise, z.B. durch Fällung des Nitriergemisches auf Eis, Filtration und Wäsche des Filtergutes mit kaltem Wasser, abgetrennt. Man fällt zweckmäßig das Gemisch auf die 12- bis 60-fache, bevorzugt 22- bis 28-fache Menge Eis. Die Temperatur stellt sich zweckmäßig in der so gebildeten Suspension auf -10 bis 0°C ein. Danach wird vorteilhaft das Dinitroisatosäureanhydrid über eine gekühlte Filtrationseinrichtung isoliert. Bevorzugt ist eine auf -10 bis +10°C, insbesondere auf -2 bis +2°C gekühlte Drucknutsche. Man wäscht das Filtergut mit kaltem Wasser, zweckmäßig von einer Temperatur von 0 bis 20°C, bevorzugt 3 bis 8°C, neutral und bläst das gekühlte Gut, zweckmäßig bei einer Temperatur von 0 bis 20°C, bevorzugt 3 bis 8°C, mit Luft oder Stickstoff trocken. Meist kann schon der feuchte Endstoff weiterverarbeitet werden.

In einer bevorzugten Ausführungsform digeriert man das kalt abgesaugte, neutral gewaschene Filtergut in einem Ausgangsstoff und Endstoff nicht lösenden, mit Wasser unendlich oder begrenzt mischbaren Lösungsmittel und trocknet es, nach erneutem Absaugen, zweckmäßig bei einer Temperatur von 20 bis 60°C, bevorzugt 35 bis 45°C. Man erhält so das an sich sehr solvolyseempfindliche 6,8-Dinitroisatosäureanhydrid in überraschend reiner Form als gelbes Kristallpulver vom Fp 165 bis 167°C. Als Lösungsmittel kommen z.B. in Frage: Nitrile wie Acetonitril, Butyronitril, Isobutyronitril, Benzonitril; Alkanole und Cycloalkanole wie Äthanol, Methanol, n-Butanol, Isobutanol, tert.-Butanol, Glykol, Glycerin, n-Propanol, Isopropanol, Amylalkohol, Cyclohexanol, 2-Methyl-4-pentanol, Äthylenglykolmonoäthyläther, 2-Äthylhexanol, Methylglykol, n-Hexanol, Isohexylalkohol, Isoheptylalkohol, n-Heptanol, Äthylbutanol, Nonylalkohol, Dodecylalkohol, Methylcyclohexanol, Diacetonalkohol, insbesondere solche mit 1 bis 4 Kohlenstoffatomen; Ketone wie Methyläthylketon, Aceton, Diisopropylketon, Diäthylketon, Methylisobutylketon, Mesityloxid, Acetophenon, Cyclohexanon, Äthylisoamylketon, Diisobutylketon, Methylcyclohexanon, Dimethylcyclohexanon; und entsprechende Gemische. Bevorzugt sind Isopropanol und n-Butanol. Zweckmäßig verwendet man das Lösungsmittel in einer Menge von 80 bis 10 000 Gewichtsprozent, vorzugsweise von 100 bis 600 Gewichtsprozent, bezogen auf Isatosäureanhydrid. Zur Verhinderung der Solvolyse ist vor der Trocknung noch ein Waschgang mit Toluol oder anderen, mit vorgenannten Alkoholen, Nitrilen und Ketonen mischbaren Lösungsmitteln zweckmäßig, um, insbesondere im Falle vorgenannter Alkohole, eine Reaktion des 6,8-Dinitroisatosäureanhydrids mit dem anhaftenden organischen Lösungsmittel zu vermeiden.

Das nach dem Verfahren der Erfindung herstellbare, neue 6,8-Dinitroisatosäureanhydrid ist ein wertvoller Ausgangsstoff für die Herstellung von Farbstoffen, Pharmazeutika und Schädlingsbekämpfungsmitteln. So kann aus ihm 2-Amino-3,5-dinitrobenzamid und aus diesem Amid durch Wasserabspaltung 3,5-Dinitro-2-amino-benzonitril hergestellt werden, das eine wertvolle Diazokomponente zur Herstellung von blauen Azofarbstoffen darstellt (DOS 16 44 141, 20 28 395, 19 05 364, 19 28 372).

Die in den folgenden Beispielen genannten Teile bedeuten Gewichtsteile.

Beispiel 1

In 1 800 Teilen 98-gewichtsprozentiger Schwefelsäure werden unter Außenkühlung bei 25°C 208 Teile 98-gewichtsprozentige Salpetersäure während 90 Minuten gegeben. Nach kurzem Rühren werden 0,5 Teile Harnstoff in kleinen Portionen zugegeben. Man kühlt das Gemisch auf 15°C und trägt bei 18°C innerhalb von einer Stunde 163 Teile Isatosäureanhydrid in das Nitriergemisch ein. Nach 3-stündigem Rühren bei 20°C gibt man das Gemisch auf 4 000 Teile Eis. Man isoliert den ausgefallenen Endstoff durch Filtration über eine auf 0°C gekühlte Drucknutsche, wäscht das Filtergut mit Wasser neutral und bläst es mit Luft trocken. Man erhält 152 Teile 6,8-Dinitroisatosäureanhydrid (60 % der Theorie) vom Fp 165 bis 167°C.

Beispiel 2

Führt man die Umsetzung analog Beispiel 1 mit anstelle der Salpetersäure 330 Teilen Kaliumnitrat durch, so erhält man die gleichen Ergebnisse.

Beispiel 3

Führt man die Umsetzung analog Beispiel 1 mit anstelle der Salpetersäure 280,5 Teilen Natriumnitrat durch, so erhält man die gleichen Ergebnisse.

Beispiel 4

Die Umsetzung wird analog Beispiel 1 durchgeführt. Nach dem Fällen auf Eis saugt man das Gemisch ab, wäscht neutral und digeriert den Filterkuchen bei 25°C in 400 Teilen Isopropanol, saugt das Gemisch erneut ab, behandelt mit 400 Teilen Toluol, saugt das Gemisch ab und trocknet bei 40°C (20 mbar). Man erhält 177 Teile 6,8-Dinitroisatosäureanhydrid (70 % der Theorie) vom Fp. 165 bis 167°C.

Beispiel 5

Führt man die Umsetzung analog Beispiel 4 durch, digeriert aber mit 400 Teilen n-Butanol statt Isopropanol, so erhält man 190 Teile 6,8-Dinitroisatosäureanhydrid (75 % der Theorie) vom Fp 165 bis 167°C.

Patentansprüche

1. 6,8-Dinitroisatosäureanhydrid.

2. Verfahren zur Herstellung von 6,8-Dinitroisatosäureanhydrid durch Nitrierung von Isatosäureanhydrid, dadurch gekennzeichnet, daß man Isatosäureanhydrid mit Salpetersäure in Gegenwart von 90- bis 100-gewichtsprozentiger Schwefelsäure bei einer Temperatur von -20 bis +80°C nitriert.

0011757

Europäisches Patentamt

**EUROPÄISCHER RECHERCHENBERICHT**

Nummer der Anmeldung
EP 79 10 4438

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | CHEMICAL ABSTRACTS, Band 63,Nr. 8, Oktober 11, 1965, Zusammenfassung Nr. 9937f linke Spalte, Columbus, Ohio, USA, G. WAGNER et al.: "Synthesis of nitrosalicoylureides and nitro-2,4-dioxodihydro-1,3-benzoxazines" & Pharm.Zentralhalle 103(11-12), 791-8 (1964) * Zusammenfassung * -- | 1 |
| D | DE - A - 2 422 239 (BASF) ---- | 1,2 |

KLASSIFIKATION DER ANMELDUNG (Int.Cl. 3)

C 07 D 265/26

RECHERCHIERTE SACHGEBIETE (Int. Cl. 3)

C 07 D 265/26

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 13-02-1980 | CHOULY |